# EUROPEAN PATENT APPLICATION

(11) **EP 1 279 407 A1**
(43) Date of publication of application: **29.01.2003**
(21) Application number: 02015504.0
(22) Date of filing: 10.07.2002
(51) Int. Cl.: A61L 9/14, B01D 53/34

(54) **Apparatus for deodorizing and sanitizing malodorous gases**

(30) Priority: 23.07.2001 IT MI20011576
(71) Applicant: Eurovix S.r.l., 25046 Cazzago San Martino (Prov. of Brescia) (IT)
(72) Inventor: Bonassi, Dario, 25046 Cazzago San Martino (Brescia) (IT)
(74) Representative: Modiano, Guido, Dr.-Ing.

(57) **Abstract**

An apparatus (1) for deodorizing and sanitizing malodorous gases comprising a reservoir (2) that has, in a lower region, a diffuser (3) for introducing a gas to be treated and, in an upper region, means (30,31) for introducing a liquid treatment product. The reservoir (2) is connected to a duct (10) for input to a dosage device (11) for the liquid treatment product controlled by a control valve (20). An air ejector (35) is further provided for sending the dosed liquid treatment product into the reservoir (2).

## Description

The present invention relates to an apparatus for deodorizing and sanitizing malodorous gases.

As is known, malodorous gases are currently deodorized and sanitized by using enzymatic liquids which are struck by the stream of air to be purified so as to remove the impurities contained in the gas.

Known kinds of solution do not allow to optimize the exchange surfaces and therefore there is a significant waste of treatment liquid; moreover, in many cases it is not possible to achieve an optimum result.

The aim of the present invention is to solve the above problem by providing an apparatus for deodorizing and sanitizing malodorous gases that offers a large surface of contact between the gases to be treated and the liquid treatment product, thus increasing the effectiveness of the treatment.

Within this aim, an object of the invention is to provide an apparatus that allows to dose precisely the quantity of liquid being treated, avoiding unnecessary waste.

Another object of the present invention is to provide an apparatus that by way of its particular constructive characteristics is capable of giving the greatest assurances of reliability and safety in use.

Another object of the present invention is to provide an apparatus that can be obtained easily starting from commonly commercially available elements and materials and is further competitive from a merely economical standpoint.

This aim and these and other objects that will become better apparent hereinafter are achieved by an apparatus for deodorizing and sanitizing malodorous gases, according to the invention, characterized in that it comprises a reservoir that has, in a lower region thereof, a diffuser for introducing a gas to be treated and, in an upper region thereof, means for introducing a liquid treatment product, said reservoir being connected to a duct for input to a dosage device for the liquid treatment product controlled by a control valve, an air ejector being further provided for sending into said reservoir the dosed liquid treatment product.

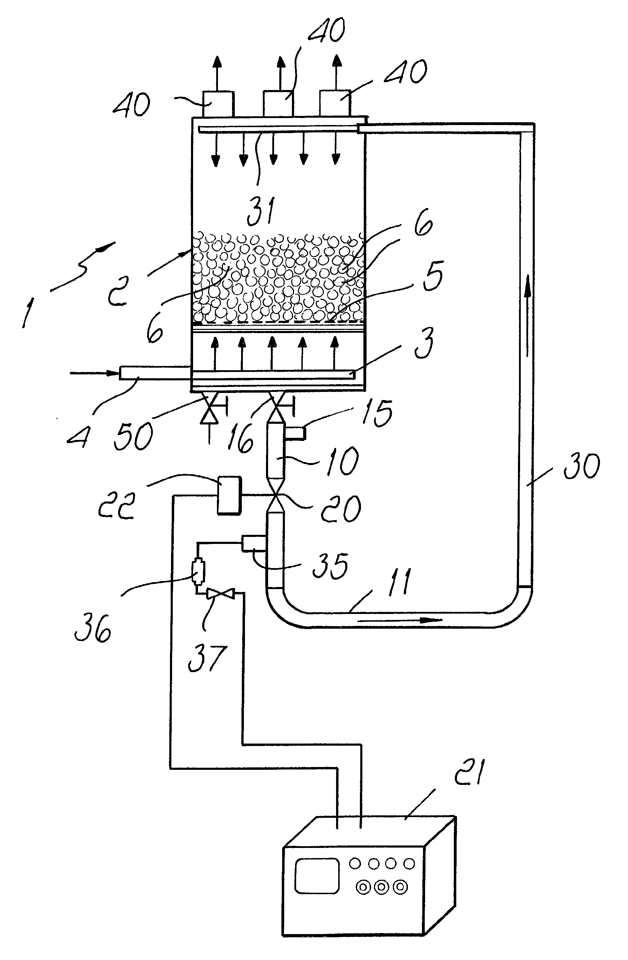

Further characteristics and advantages of the present invention will become better apparent from the following detailed description of a preferred but not exclusive embodiment of an apparatus for deodorizing and sanitizing malodorous gases, illustrated by way of non-limitative example in the accompanying drawing, wherein the sole figure is a diagram of the apparatus.

With reference to the figure, the apparatus for deodorizing and sanitizing malodorous gases according to the invention, generally designated by the reference numeral 1, comprises a reservoir 2 which has, in its lower part, a diffuser 3 for introducing the air to be treated, which arrives from an input duct designated by the reference numeral 4.

Above the region affected by the diffuser 3 there is a separation grille 5, above which there is a device for increasing the exchange surface between the enzymatic liquid treatment product and the air to be treated, which is provided by means of a plurality of bodies 6 that form an amorphous mass that is permeable to the air stream and can be struck by the stream of gas to be treated.

A duct 10 for introducing the product to be treated into a dosage device 11 is provided in the lower part of the reservoir.

The input duct is controlled by a control valve 20, which is controlled by a central unit and is driven by a pneumatic valve 22 controlled by the central unit.

Advantageously, on the duct 10 there is an inspection filter 15 and there is a manually operated gate valve 16.

The dosage device 11 in which the quantity of liquid dosed according to the opening time of the control valve 20 is collected is substantially U-shaped and is formed on a liquid input duct, designated by the reference numeral 30, which ends inside the upper part of the reservoir 2 at means for introducing the enzymatic treatment liquid, which are provided by liquid diffusers 31 located in the upper part of the reservoir.

Downstream of the control valve 20 in the direction of the flow of liquid there is an air ejector 35, which is also controlled by the central unit 21 and is arranged on an air input duct on which a flow regulator 36 and a check valve 37 are provided.

At the upper part of the reservoir 2 there are also discharge openings 40 for the outflow of the treated air.

The apparatus according to the invention can be actuated manually or automatically, with process management derived from the central unit 21.

In practical operation, the air to be treated is introduced in the reservoir by means of the intake 4, and by exiting from the diffuser 3 into the reservoir it propagates upward, striking the bodies 6, which provide a large contact surface between the malodorous air to be treated and the enzymatic liquid product that descends in contercurrent.

The liquid has been dosed beforehand by opening the control valve 20, whose opening time may have been adjusted manually or automatically.

Once the liquid has been dosed, the ejector 35 is actuated and brings the product into the reservoir; the liquid product, by gravity, descends and encounters the bodies 6, which are wet by the product.

As its descent continues, the product collects on the bottom of the reservoir 1 to be used again.

To eliminate the spent liquid product after a preset utilization time, there is a discharge valve 50, which conveys the product outside.

From the above description it is thus evident that the invention achieves the intended aim and objects, and in particular the fact is stressed that an apparatus is provided which allows to handle automatically the deodorization and sanitizing of malodorous gases, providing a very large exchange surface by way of the presence of the bodies 6, which are affected by the liquid beforehand.

The invention thus conceived is susceptible of numerous modifications and variations, all of which are within the scope of the inventive concept.

All the details may further be replaced with other technically equivalent elements.

In practice, the materials used, as well as the contingent shapes and dimensions, may be any according to requirements.

The disclosures in Italian Patent Application No. MI2001A001576 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An apparatus (1) for deodorizing and sanitizing malodorous gases, **characterized in that** it comprises a reservoir (2) that has, in a lower region thereof, a diffuser (3) for introducing a gas to be treated and, in an upper region thereof, means (30,31) for introducing a liquid treatment product, said reservoir being connected to a duct (10) for input to a dosage device (11) for the liquid treatment product controlled by a control valve (20), an air ejector (35) being further provided for sending into said reservoir the dosed liquid treatment product.

2. The apparatus according to claim 1, **characterized in that** it comprises, inside said reservoir (2), above the region affected by said diffuser (3), a grille (5) for separation of the inside of said reservoir.

3. The apparatus according to the preceding claims, **characterized in that** it comprises, above said separation grille (5), a device for increasing the exchange surface between said liquid treatment product and the air to be treated.

4. The apparatus according to claim 3, **characterized in that** said device for increasing the exchange surface between the liquid treatment product and the air to be treated is constituted by a plurality of bodies (6) that form an amorphous mass having a large surface.

5. The apparatus according to one or more of the preceding claims, **characterized in that** it comprises a central unit (21) that drives a pneumatic valve (22) for actuating said control valve (20).

6. The apparatus according to one or more of the preceding claims, **characterized in that** it comprises, on the duct (10) for input to the dosage device (11), an inspection filter (15) and gate valve (16) with manual actuation.

7. The apparatus according to one or more of the preceding claims, **characterized in that** said dosage device (11) is substantially U-shaped and is formed on the duct (30) for introducing liquid in said reservoir.

8. The apparatus according to one or more of the preceding claims, **characterized in that** said air ejector (35) is controlled by said central unit (21).

9. The apparatus according to one or more of the preceding claims, **characterized in that** it comprises, at the upper part of said reservoir, discharge openings (40) for the outflow of the treated air.

10. The apparatus according to one or more of the preceding claims, **characterized in that** it comprises, on the bottom of said reservoir, a valve (50) for discharging the spent liquid treatment product.
